# EUROPÄISCHE PATENTANMELDUNG

(11) **EP 3 549 931 A1**
(43) Veröffentlichungstag der Anmeldung: **09.10.2019**
(21) Anmeldenummer: 18166140.6
(22) Anmeldetag: 06.04.2018
(51) Int. Cl.: C07C 263/20, F26B 11/12, F26B 11/16

(54) **VERFAHREN ZUR RÜCKGEWINNUNG VON DIISOCYANATEN AUS DESTILLATIONSRÜCKSTÄNDEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die vorliegende Erfindung betrifft ein Verfahren zur Rückgewinnung von monomeren bei Raumtemperatur festen Diisocyanaten aus einem Destillationsrückstand, umfassend die folgenden Schritte:
(i) Bereitstellen mindestens eines bei Raumtemperatur feste Diisocyanate enthaltenden Rückstands und
(ii) Trennung des Rückstands in mindestens einem Knetertrockner, Schaufeltrockner und/oder Walzentrockner in Gegenwart von weniger als 2 Gew.-% Bitumen, bezogen auf die Masse des in Schritt (i) bereitgestellten Rückstands, in einen gasförmigen Teil, enthaltend monomeres bei Raumtemperatur festes Diisocyanat, und einen spröden, an bei Raumtemperatur festem Diisocyanat abgereicherten Rückstand.

## Beschreibung

Die vorliegende Erfindung betrifft ein Verfahren zur Rückgewinnung von monomeren bei Raumtemperatur festen Diisocyanaten aus einem Destillationsrückstand. Außerdem betrifft die Erfindung ein monomeres bei Raumtemperatur festes Diisocyanat, erhältlich nach diesem Verfahren sowie die Verwendung eines Knetertrockners, Schaufeltrockners oder Walzentrockners zur Versprödung eines bei Raumtemperatur feste Diisocyanate enthaltenden Rückstands. Auch betrifft die Erfindung eine Zusammensetzung, enthaltend mindestens ein erfindungsgemäßes, monomeres bei Raumtemperatur festes Diisocyanat sowie aus dieser Zusammensetzung erhältliche Elastomere.

Die großtechnische Herstellung von Diisocyanaten durch Umsetzung von Aminen mit Phosgen in Lösungsmitteln ist bekannt und in der Literatur ausführlich beschrieben (Ullmanns Encyklopädie der technischen Chemie, 4. Auflage, Band 13, Seite 347-357, Verlag Chemie, GmbH, D-6940 Weinheim, 1977 oder auch EP 1 575 908 A1). Zumeist fällt bei der Herstellung reiner destillierter Diisocyanate in den Destillationsprozessen ein Nebenproduktstrom an, der nach weitmöglicher destillativer Abtrennung von freien lsocyanaten als Rückstand entsorgt werden muss. Dieser Rückstand enthält, abhängig von der chemischen Natur des hergestellten Diisocyanats, einen beträchtlichen Anteil an monomerem Diisocyanat. Wünschenswert war es also, das monomere Diisocyanat aus dem Rückstand bestmöglich zurückgewinnen zu können, um die Gesamtausbeute steigern zu können, ohne dass die Gesamtwirtschaftlichkeit des Verfahrens verschlechtert wird.

Die WO 2007/036479 A1 offenbart ein allgemeines Verfahren zur Aufreinigung von isocyanathaltigen Rückständen, bei dem weniger bevorzugt auch Naphthyldiisocyanat eingesetzt werden kann. Ausführungsbeispiele werden nicht offenbart. Die Destillation findet bei Temperaturen zwischen 210 °C und 330 °C statt, wobei eine hochviskose Flüssigkeit und/oder ein nicht versprödender, also pastöser Feststoff entsteht, die/der unter den im Apparat herrschenden Bedingungen fließfähig ist. Je nach Rückstand kann unter den genannten Prozessbedingungen eine Oligomerisierung der enthaltenen Isocyanate nicht ausgeschlossen werden, so dass letztlich doch das Risiko einer Versprödung besteht, für die die Apparate nicht ausgelegt sind.

Die EP 0 626 368 A1 beschreibt ein Verfahren zur Herstellung von reinen, destillierten Isocyanaten bei dem der Rückstand mit 2 bis 50 Gew.-% hochsiedenden Kohlenwasserstoffen versetzt wird und das Isocyanat aus diesem Rückstand bei Temperaturen von 160 °C bis 280 °C extrahiert wird. Bei diesem Verfahren werden Knetertrockner eingesetzt, die eine relativ aufwendige Technik mit mechanisch bewegten Teilen bedeuten. Da bei dieser Technik der kontinuierliche Austrag von rieselfähigem Material gewährleistet sein muss, ist die Beschaffenheit des Rückstandes ausschlaggebend und schränkt die Nutzung stark ein. Nachteilig ist auch die Handhabung und Entsorgung größerer Mengen eines ansonsten verfahrensfremden Hilfsstoffs.

Die US 3,694,323 offenbart ein Verfahren zur Rückgewinnung eines Isocyanates aus seinem Phosgenierungsrückstand unter Zuhilfenahme eines so genannten Isocyanat-Austausch-Mittels, welches einen höheren Siedepunkt als das zu reinigende Isocyanat aufweist und somit die Viskosität des Phosgenierungsrückstand erniedrigt und eine Reinigung ermöglicht. Nachteilig an diesem Verfahren ist jedoch, dass das gereinigte Isocyanat mit dem Isocyanat-Austausch-Mittel verunreinigt wird wie schon in EP 0 626 368 A1 zusätzlicher Aufwand für die Handhabung und Entsorgung des Isocyanat-Austausch-Mittels anfällt.

Die DE10260092A1 beschreibt ein Verfahren zur Reinigung von Isocyanaten, wobei in einem Destillationsschritt ein Rückstandsstrom anfällt, der nicht verdampfbaren Rückstand und Isocyanat enthält. Dieser wird in einem Schritt c) in einen weiteren, isocyanathaltigen Brüdenstrom und einen im Wesentlichen nicht verdampfbaren Rückstand enthaltenden Strom aufgetrennt. Die weiteren Eigenschaften dieses Rückstands sind mit hochviskos oder fest umschrieben, wobei nicht angegeben ist, unter welchen Bedingungen dies der Fall ist. Die Auswahl der empfohlenen Trocknungsapparate sowie das Ausführungsbeispiel anhand von Toluylendiisocyanat lassen darauf schließen, dass im Trockner eine fließfähige, viskose oder hochviskose Phase vorliegen muss, da die meisten genannten Apparate für den Anfall von Feststoffen nicht geeignet sind.

Im zitierten Stand der Technik erfolgt die Abtrennung also entweder unter Zuhilfenahme größerer Mengen an Zusatzmitteln, die eine Versprödung des Rückstands hin zu einem rieselfähigen Rückstand ermöglichen oder durch eine Abkühlung des Rückstands schon im Trocknungsapparat mit den oben beschriebenen Nachteilen. Alternativerfolgt die Trennung unter solchen Bedingungen, dass der Rückstand zumindest pastös und förderfähig bleibt.

Überraschend wurde nun gefunden, dass sich Destillationsrückstände von bei Raumtemperatur festen Diisocyanaten in besonderer Weise eignen, um diese auch in Abwesenheit von Bitumen in einem Knetertrockner, Schaufeltrockner oder Walzentrockner in einen gasförmigen, monomeres Diisocyanat enthaltenden Teil und einen spröden, an monomerem bei Raumtemperatur festem Diisocyanat abgereicherten Rückstand aufzutrennen.

Gegenstand der Erfindung ist deshalb ein Verfahren zur Rückgewinnung von monomeren bei Raumtemperatur festen Diisocyanaten aus einem Destillationsrückstand, umfassend die folgenden Schritte:
(i) Bereitstellen mindestens eines bei Raumtemperatur feste Diisocyanate enthaltenden Rückstands und
(ii) Trennung des Rückstands in einem Knetertrockner, Schaufeltrockner und/oder Walzentrockner in Gegenwart von weniger als 2 Gew.-% Bitumen, bezogen auf die Masse des in Schritt (i) bereitgestellten Rückstands, in einen gasförmigen Teil, enthaltend monomeres bei Raumtemperatur festes Diisocyanat, und einen spröden, an monomerem bei Raumtemperatur festem Diisocyanat abgereicherten Rückstand.

Vorliegend wird unter dem Begriff "spröde" für die Bezeichnung von Stoffen, wie z.B. des an monomerem bei Raumtemperatur festem Diisocyanat abgereicherten Rückstands, verwendet, wenn die Stoffe im Spannungs-Dehnungs-Diagramm, das man erhält, wenn man eine Probe mit einer Zugkraft F belastet und die verursachte Längenänderung ΔL darüber aufträgt, eine steile Hook'sche Gerade aufweisen, die den proportionalbereich von Dehnung und Spannung kennzeichnet, wobei die Hook'sche Gerade mit dem Bruch endet.

In einer bevorzugten Ausführungsform des erfindungsgemäßen Verfahrens ist der Knetertrockner, Schaufeltrockner oder Walzentrockner ohne Kühlzone ausgeführt. Auf diese Weise werden interne Wärmeverluste vermieden und die Wahrscheinlichkeit für Leckagen des jeweiligen Trockners vermindert.

Vorliegend wird unter dem Begriff "Kühlzone" verstanden, dass der Trockner einen Bereich umfasst, in dem die Wandtemperatur unabhängig vom Rest des Trockners auf eine niedrigere Temperatur geregelt werden kann. Dies soll die Verfestigung des Rückstands unterstützen, ist aber mit oben genannten Nachteilen verbunden.

In einer weiteren bevorzugten Ausführungsform des Verfahrens erfolgt die Trennung des Rückstandes in Schritt (ii) in einem Knetertrockner oder einem Walzentrockner und besonders bevorzugt in einem Walzentrockner erfolgt. Diese sind in besonderer Weise geeignet, versprödende Gemische zu verarbeiten. Insbesondere Walzentrockner haben den Vorteil, dass aufgrund ihrer Konstruktion eine Versprödung in sehr kurzer Zeit erfolgt. Grundsätzlich beruht die Versprödung im Wesentlichen auf zwei Effekten, dem Entzug von Diisocyanat durch Verdampfung und der Oligomerisierung von Diisocyanat. Gerade bei Walzentrockner ist der Beitrag der Verdampfung von Diisocyanat gegenüber anderen Trocknern erhöht, was zu einer höheren Ausbeute des Wertstoffs führt.

In einer besonders bevorzugten Ausführungsform des Verfahrens erfolgt die Trennung in Schritt (ii) in Gegenwart von ≤ 1 Gew.-% Bitumen, bevorzugt in Abwesenheit von Bitumen.

In einer weiteren bevorzugten Ausführungsform des Verfahrens liegt die Temperatur in Schritt (ii) bei ≥ 130 °C bis ≤ 270 °C, bevorzugt bei ≥ 140°C bis ≤ 200 °C und besonders bevorzugt bei ≥ 150 °C bis ≤ 190 °C. In diesem Temperaturbereich erfolgt einerseits eine Verdampfung oder Sublimation von monomerem Diiosocyanat aus dem Rückstand, so dass dieses zurückgewonnen werden kann. Andererseits erfolgt bereits in begrenztem Umfang eine Oligomerisierung der Diisocyanate, die zur gewünschten Versprödung des Rückstands beiträgt ohne prohibitive Ausbeuteverluste zu bewirken.

In einer weiteren bevorzugten Ausführungsform des liegt der Druck in Schritt (ii) bei ≥ 0,1 mbar bis ≤ 1020 mbar, bevorzugt bei ≥ 0,5 mbar bis ≤ 25 mbar und besonders bevorzugt bei ≥ 1 mbar bis ≤ 10 mbar. Dieser bevorzugte Druckbereich unterstützt eine rasche Entfernung des monomeren Diisocyanats, so dass die Verweilzeiten im entsprechenden Trockner kurz gehalten werden können. Erheblich geringere Drücke sind nicht förderlich, da diese die Kondensation des in Schritt (ii) gasförmig anfallenden Teils zur weiteren Verwendung erschweren.

Geeignete bei Raumtemperatur feste Diisocyanate sind 1,5-Naphthalindiisocyanat, 1,8-Naphthalindiisocyanat, 1,4-Phenylendiisocyanat, Tetralindiisocyanat, o-Tolidindiisocyanat, Duroldiisocyanat, Benzidindiisocyanat und/oder 1,4-Anthrylendiisocyanat.

In einer weiteren bevorzugten Ausführungsform des Verfahrens ist das bei Raumtemperatur feste Diisocyanat bevorzugt 1,5-Naphthalindiisocyanat, 1,8-Naphthalindiisocyanat oder 1,4-Phenylendiisocyanat, besonders bevorzugt 1,5-Naphthalindiisocyanat oder 1,4-Phenylendiisocyanat und ganz besonders bevorzugt 1,5-Naphthalindiisocyanat. Diese Isocyanate zeichnen sich dadurch aus, dass sie eine erhöhte Reaktivität aufweisen, was zu einer zuverlässigen Versprödung des Rückstands beiträgt.

Geeignete Knetertrockner, Schaufeltrockner oder Walzentrockner sind insbesondere solche Trockner, die Einrichtungen für die Abreinigung der bewegten Teile, sowie des Trocknergehäuses aufweisen. Dies gilt insbesondere für die beheizten Oberflächen. Besonders bevorzugt dienen Schabmesser und/oder Gegenhaken zur Ablösung des versprödeten Rückstands.

Es eignen sich beispielsweise Vakuum-Walzentrockner mit einer Walze oder bevorzugt Vakuum-Doppelwalzentrockner. Es handelt sich dabei um sogenannte Dünnschichttrockner, bei denen das zu trocknende Gut auf langsam rotierende, meist dampfbeheizte Walzen aufgetragen wird. Dabei verdampfen Anteile des zu trocknenden Gutes und der verbleibende Rückstand wird von der oder den Walzen abgeschabt. Aufgrund der großen Oberfläche für die konduktive Wärmeübertragung reichen recht kurze und damit schonende Verweilzeiten für die Trocknung aus. Beim Betrieb im Vakuum erfolgt der Austrag des nicht verdampften Rückstands entweder über entsprechende Vakuumschleusen oder der Betrieb erfolgt chargenweise wobei die Kammer des Trockners zwischenzeitlich auf Atmosphärendruck gebracht wird, um das getrocknete Gut auszutragen.

Geeignete Knetertrockner sind beispielsweise sogenannte ein- oder zweiwellige Kneterreaktoren, mit großen Heizflächen und Werkzeugen zum Kneten und Durchmischen des zu trocknenden Guts. Die Knetwerkzeuge sind dabei so auf der oder den Wellen des Trockners angeordnet, dass sie einer einerseits für eine radiale Durchmischung und andererseits für einen axialen Transport des Trockenguts sorgen. Gleichzeitig Bei zweiwelligen Apparaten können die Wellen je nach Anforderung gleich- oder auch gegenläufig betrieben werden. Die Spaltmaße sind bevorzugt so gewählt, dass die beheizten Oberflächen durch die Bewegung der Knetwerkzeuge selbsttätig gereinigt werden. Die intensive Durchmischung führt zu einer schnellen Verdamfpung der verdampfbaren Anteile und zu einem zusätzlichen Energieeintrag in das zu trocknende Gut.

Geeignete Schaufeltrockner bestehen beispielsweise aus einem beheizten Gehäuse, in dem sich ein oder mehrere meist ebenfalls beheizte Wellen befinden. Auf den Wellen sind Schaufeln derart angeordnet, dass das zu trocknende Gut wiederum radial durchmischt und gleichzeitig axial gefördert wird. Dies wird beispielsweise durch eine Schrägstellung der Schaufeln erreicht, so dass durch eine Änderung der Drehrichtung auch eine Änderung der axialen Förderrichtung erreicht wird, was zum Austrag des getrockneten Guts genutzt werden kann.

Bevorzugt beträgt die mittlere Verweilzeit des Rückstands im Knetertrockner, Schaufeltrockner oder Walzentrockner von ≥ 0,5 Minuten bis ≤ 4 Stunden, besonders bevorzugt von ≥ 0,5 Minuten bis ≤ 60 Minuten, ganz besonders bevorzugt von ≥ 0,5 bis ≤ 15 Minuten und am meisten bevorzugt von ≥ 1 bis ≤ 10 Minuten. Die letztlich erforderliche Verweilzeit, die einen Kompromiss aus zuverlässiger Versprödung, hoher Rückgewinnung von monomerem Diisocyanat und begrenzter Apparategröße darstellt, hängt dabei vom gewählten Isocyanat, dem Druck und der Temperatur im Trockner ab und kann vom Fachmann in einer Versuchsreihe ermittelt werden.

Der abgelöste Rückstand kann bevorzugt halbkontinuierlich, also taktweise ausgetragen und anschließend abgekühlt werden. In einer besonders bevorzugten Ausführungsform erfolgt der Austrag aus dem Apparat über eine Vakuumschleuse.

In einer weiteren bevorzugten Ausführungsform des Verfahrens werden die Bedingungen im Schritt (ii) so gewählt, dass der in Schritt (ii) erhaltene, spröde Rückstand ≤ 5 Gew.-%, bevorzugt ≤ 2 Gew.-%, besonders bevorzugt ≤ 0,2 Gew.-% und ganz besonders bevorzugt < 0,1 Gew.-% an monomerem, bei Raumtemperatur festem Diisocyanat bezogen auf die Gesamtmasse des Rückstands enthält. Dies ist besonders vorteilhaft, da höhere Konzentrationen an monomerem Diisocyanat einerseits einen Verlust an Wertstoff bedeuten, andererseits die monomeren Diisocyanate teilweise gesundheitsschädliche oder korrosive Eigenschaften aufweisen.

Prinzipiell kann die Herstellung der bei Raumtemperatur festen Diisocyanate auf beliebigen Wegen, beispielsweise durch Umsetzung der entsprechenden Diamine oder deren Salze mit Phosgen, erfolgen. Wichtig ist jeweils nur, dass bei dem genutzten Verfahren mindestens ein, bei Raumtemperatur feste Diisocyanate enthaltender Rückstand übrig bleibt, der dann im erfindungsgemäßen Schritt (i) eingesetzt werden kann.

In einer weiteren bevorzugten Ausführungsform des Verfahrens stammt der in Schritt (i) bereitgestellte, bei Raumtemperatur feste Diisocyanate enthaltende, Rückstand aus der Destillation eines durch Phosgenierung der entsprechenden Diamine hergestellten Diisocyanats. Besonders bevorzugt ist es, dass der in Schritt (i) bereitgestellte bei Raumtemperatur feste Diisocyanate enthaltende Rückstand aus der Phosgenierung der entsprechenden Diamine, bevorzugt aus der Flüssigphasen-Phosgenierung der entsprechenden Diamine kommt.

Vorliegend ist unter dem Begriff "entsprechendes Diamin" jeweils das herzustellende bei Raumtemperatur feste Diisocyanat zu verstehen, bei dem die beiden Isocyanatgruppen gegen Aminogruppen ausgetauscht sind. Beispielhaft sei 1,5-Naphthalindiamin als entsprechendes Diamin zum 1,5-Naphthalindiisocyanat genannt. Wenn das bei Raumtemperatur feste Diisocyanat ein Isomerengemisch ist, kommt eine entsprechende Isomerenmischung an Diaminen zum Einsatz.

Wie aus dem erfindungsgemäßen Verfahren ersichtlich, lassen sich Knetertrockner, Schaufeltrockner und/oder Walzentrockner sehr gut zur Abreicherung von monomeren bei Raumtemperatur festen Diisocyanaten aus einem bei Raumtemperatur feste Diisocyanate enthaltenden Rückstand und/oder Versprödung eines bei Raumtemperatur feste Diisocyanate enthaltenden Rückstands verwenden.

Ein weiterer Gegenstand der Erfindung ist ein monomeres bei Raumtemperatur festes Diisocyanat, erhalten oder erhältlich nach dem erfindungsgemäßen Verfahren. Durch den weitestgehenden Verzicht auf Hilfsstoffe wie Bitumen in Schritt (ii) des Verfahrens wird auch die Kontamination des aus dem Rückstand zurückgewonnenen, monomeren bei Raumtemperatur festen Diisocyanats mit diesen Hilfsstoffen minimiert oder gar gänzlich vermieden.

Außerdem ist ein weiterer Gegenstand der Erfindung ist die Verwendung eines Knetertrockners, Schaufeltrockners und/oder Walzentrockners zur Minimierung des Hilfsstoffeinsatzes bei der Versprödung eines bei Raumtemperatur feste Diisocyanate enthaltenden Rückstands. Bei dieser Verwendung handelt es sich bei dem bei Raumtemperatur feste Diisocyanate enthaltenden Rückstand bevorzugt um einen Destillationsrückstand.

Die nach dem erfindungsgemäßen Verfahren erhaltenen, monomeren bei Raumtemperatur festen Diisocyanate können vielfältigen Verwendungszwecken zugeführt werden. Insbesondere ist die Weiterverarbeitung mit NCO-reaktiven Verbindungen wie Polyolen zu Polyurethanen, ggf. über Präpolymere als Zwischenstufen, zu nennen.

Diese Polyurethane haben bevorzugt Rohdichten von 200 kg/m³ bis 1400 kg/m³, besonders bevorzugt von 600 kg/m³ bis 1400 kg/m³ und ganz besonders bevorzugt von 800 kg/m³ bis 1400 kg/m³. Ganz besonders bevorzugt werden zellige oder massive Gießelastomere hergestellt, ganz besonders bevorzugt Gießelastomere auf Polyesterpolyol Basis.

Somit ist ein weiterer Gegenstand der vorliegenden Erfindung eine Zusammensetzung enthaltend mindestens erfindungsgemäßes monomeres bei Raumtemperatur festes Diisocyanat und mindestens eine NCO-reaktive Verbindung, bevorzugt mindestens ein Polyesterpolyol.

Die Zusammensetzung kann darüber hinaus übliche Hilfs- und Zusatzmittel, wie z.B. Rheologieverbesserer (zum Beispiel Ethylencarbonat, Propylencarbonat, dibasische Ester, Zitronensäureester), Stabilisatoren (zum Beispiel Broenstedt- und Lewis-Säuren, wie etwa Salzsäure, Phosphorsäure, Benzoylchlorid, Organomineralsäuren wie Dibutylphosphat, weiterhin Adipinsäure, Äpfelsäure, Bernsteinsäure, Traubensäure oder Zitronensäure), UV-Schutzmittel (zum Beispiel 2,6-Dibutyl-4-methylphenol), Hydrolyseschutzmittel (zum Beispiel sterisch gehinderte Carbodiimide), Emulgatoren sowie Katalysatoren (zum Beispiel Trialkylamine, Diazabicyclooctan, Zinndioctoat, Dibutylzinndilaurat, N-Alkylmorpholin, Blei-, Zink-, Zinn-, Kalzium-, Magnesiumoctoat, die entsprechenden Naphthenate und p-Nitrophenolat und/oder auch Quecksilberphenylneodecanoat) und Füllstoffe (zum Beispiel Kreide), gegebenenfalls in das/den später zu bildende/n Polyurethan/Polyharnstoff einbaufähige Farbstoffe (die also über Zerewitinoff-aktive Wasserstoffatome verfügen) und/oder Farbpigmente enthalten.

Als NCO-reaktive Verbindungen können alle dem Fachmann bekannten Verbindungen eingesetzt werden.

Als NCO-reaktive Verbindungen können Polyetherpolyole, Polyesterpolyole, Polycarbonatpolyole und Polyetheramine, welche eine mittlere OH- bzw. NH-Funktionalität von mindestens 1,5 aufweisen, sowie kurzkettige Polyole und Polyamine (Kettenverlängerer oder Vernetzer), wie sie aus dem Stand der Technik hinlänglich bekannt sind. Dies können beispielsweise niedermolekulare Diole (z.B. 1,2-Ethandiol, 1,3- bzw. 1,2-Propandiol, 1,4-Butandiol), Triole (z.B. Glycerin, Trimethylolpropan) und Tetraole (z.B. Pentaerythrit) sein, aber auch höhermolekulare Polyhydroxyverbindungen wie Polyetherpolyole, Polyesterpolyole, Polycarbonatpolyole, Polysiloxanpolyole, Polyamine und Polyetherpolyamine sowie Polybutadienpolyole.

Polyetherpolyole sind in an sich bekannter Weise durch Alkoxylierung von geeigneten Starter-Molekülen unter Basenkatalyse oder Einsatz von Doppelmetallcyanidverbindungen (DMC-Verbindungen) zugänglich. Geeignete Starter-Moleküle für die Herstellung von Polyetherpolyolen sind beispielsweise einfache, niedermolekulare Polyole, Wasser, organische Polyamine mit mindestens zwei N-H-Bindungen oder beliebige Gemische derartiger Starter-Moleküle. Bevorzugte Starter-Moleküle zur Herstellung von Polyetherpolyolen durch Alkoxylierung, insbesondere nach dem DMC-Verfahren, sind insbesondere einfache Polyole wie Ethylenglykol, Propylenglykol-1,3- und Butandiol-1,4, Hexandiol-1,6, Neopentylglykol, 2-Ethylhexandiol-1,3, Glyzerin, Trimethylolpropan, Pentaerythrit sowie niedermolekulare, Hydroxylgruppen aufweisende Ester derartiger Polyole mit Dicarbonsäuren der nachstehende beispielhafte genannten Art oder niedermolekulare Ethoxylierungs- oder Propoxylierungsprodukte derartiger einfacher Polyole oder beliebige Gemische derartiger modifizierter oder nicht modifizierter Alkohole. Für die Alkoxylierung geeignete Alkylenoxide sind insbesondere Ethylenoxid und/oder Propylenoxid, die in beliebiger Reihenfolge oder auch im Gemisch bei der Alkoxylierung eingesetzt werden können.

Polyesterpolyole können in bekannter Weise durch Polykondensation von niedermolekularer Polycarbonsäurederivaten, wie beispielsweise Bernsteinsäure, Adipinsäure, Korksäure, Azelainsäure, Sebacinsäure, Dodekandisäure, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, Dimerfettsäure, Trimerfettsäure, Phthalsäure, Phthalsäureanhydrid, Isophthalsäure, Terephthalsäure, Zitronensäure oder Trimellithsäure, mit niedermolekularen Polyolen, wie beispielsweise Ethylenglykol, Diethylenglykol, Neopentylglykol, Hexandiol, Butandiol, Propylenglykol, Glycerin, Trimethylolpropan 1,4-Hydroxymethylcyclohexan, 2-Methyl-1,3-propandiol, Butantriol-1,2, 4, Triethylenglykol, Tetraethylenglykol, Polyethylenglykol, Dipropylenglykol, Polypropylenglykol, Dibutylenglykol und Polybutylenglykol, oder durch Ring öffnende Polymerisation cyclischer Carbonsäureester, wie ε-Caprolacton, herstellen. Darüber hinaus lassen sich auch Hydroxycarbonsäurederivate, wie beispielsweise Milchsäure, Zimtsäure oder ω-Hydroxycapronsäure zu Polyesterpolyolen polykondensieren. Es können aber auch Polyesterpolyole oleochemischer Herkunft verwendet werden. Derartige Polyesterpolyole können beispielsweise durch vollständige Ringöffnung von epoxidierten Triglyceriden eines wenigstens teilweise olefinisch ungesättigte Fettsäure-enthaltenden Fettgemisches mit einem oder mehreren Alkoholen mit 1 bis 12 C-Atomen und anschließender partieller Umesterung der Triglycerid-Derivate zu Alkylesterpolyolen mit 1 bis 12 C-Atomen im Alkylrest hergestellt werden.

Die NCO-reaktive Verbindung kann als Vernetzerkomponente bzw. Kettenverlängerer kurzkettige Polyole bzw. Polyamine enthalten. Typische Kettenverlängerer sind Diethylentoluoldiamin (DETDA), 4,4'-Methylenbis-(2,6-diethyl)-anilin (MDEA), 4,4'-Methylenbis-(2,6-diisopropyl)-anilin (MDIPA), 4,4'-Methylen-bis-(3-chloro-2,6-diethyl)-anilin (MCDEA), Dimethylthiotoluoldiamin (DMTDA, Ethacure® 300), N,N'-Di(sec-butyl)-amino-biphenylmethan (DBMDA, Unilink® 4200) oder N,N'-Di-sec-butyl-p-phenylendiamin (Unilink® 4100), 3,3'-Dichloro-4,4'-diaminodiphenylmethan (MBOCA), Trimethylenglykol-di-p-aminobenzoat (Polacure 740M). Aliphatische aminische Kettenverlängerer können ebenfalls eingesetzt oder mitverwendet werden. 1,3-Propandiol, 1,4-Butandiol, 2,3-Butandiol, 1,5-Pentandiol, 1,6-Hexandiol und HQEE (Hydrochinon-di(β-hydroxyethyl)ether) sowie Wasser. Ganz besonders bevorzugt wird 1,4-Butandiol für massive Gießelastomere und Wasser für zellige Gießelastomere verwendet.

Eine Übersicht über Polyurethane, ihre Eigenschaften und Anwendungen wird beispielsweise im Kunststoffhandbuch, Band 7, Polyurethane, 3. neu bearbeitete Auflage, Band 193, herausgegeben von Prof. Dr. G.W. Becker und Prof. Dr. D. Braun (Carl-Hanser-Verlag, München, Wien) gegeben.

Bevorzugt werden NCO-terminierte Prepolymere mit einem NCO-Gehalt von 2 bis 15 Gew.-%, ganz besonders von 2-10 Gew.-% eingesetzt. Das bei Raumtemperatur feste Diisocyanat wird bevorzugt mit Polyolen der Funktionalität 2 bis 3 bevorzugt 2 und einer OH-Zahl von 28-112 mg KOH/g Substanz zu Prepolymeren umgesetzt. Bevorzugt werden Ester basierte Polyole eingesetzt. Die so hergestellten NCO-Prepolymere werden entweder direkt weiter umgesetzt oder als lagerstabile Prepolymere in beispielsweise Fässern bis zu ihrer endgültigen Verwendung gelagert. Bevorzugt werden 1,5-NDI basierte Prepolymere eingesetzt. Die Herstellung der Gießelastomere (Formteile) wird vorteilhaft bei einem NCO/OH Verhältnis von 0,7 bis 1,30 durchgeführt. Im Falle von zelligen Elastomeren wird die Menge des in das Formwerkzeug eingebrachten Gemisches üblicherweise so bemessen, dass die erhaltenen Formkörper die bereits dargestellte Dichte aufweisen. Die Ausgangskomponenten werden üblicherweise mit einer Temperatur von 30 bis 110 °C in das Formwerkzeug eingebracht. Die Verdichtungsgrade liegen zwischen 1,1 und 8, vorzugsweise zwischen 2 und 6. Die zelligen Elastomere werden zweckmäßigerweise mit einer Niederdruck-Technik oder insbesondere der Reaktionsspritztechnik (RIM) in offenen, bevorzugt geschlossenen Formwerkzeugen hergestellt.

Die Reaktionsspritzguß-Technik wird beispielsweise beschrieben von H. Piechota und H. Röhr in "Integral Schaumstoffe", Carl Hanser-Verlag, München, Wien 1975; D.J. Prepelka und J.L. Wharton in Journal of Cellular Plastics, März/April 1975, Seiten 87 bis 98 und U. Knipp in Journal of CellularPlastics, März/April 1973, Seiten 76-84.

Zusatzstoffe wie Rizinusöl oder Carbodiimide (bspw. Stabaxole der Rheinchemie als Hydrolyseschutzmittel, 2,2',6,6'-Tetraisopropyldiphenylcarbodiimid ist ein bekannter Vertreter) können sowohl dem Polyol als auch dem Prepolymer zugesetzt werden. Wasser, Emulgatoren, Katalysatoren und/oder Hilfs- und/oder Zusatzstoffe bilden mit dem Polyol gängigerweise die Polyolkomponente.

Zur besseren Entformung ist es üblich die Formwerkzeuge mit äußeren Trennmitteln zu versehen, beispielsweise Verbindungen auf Wachs- oder Silikonbasis oder wässrige Seifenlösungen. Die entformten Formkörper werden üblicherweise 1 bis 48 Stunden bei Temperaturen von 70 bis 120°C nachgetempert.

Als Emulgator werden beispielsweise sulfonierte Fettsäuren sowie weitere allgemein bekannte Emulgatoren eingesetzt, wie z. B. Polyglykolester von Fettsäuren, Alkylarylpolyglykolether, Alkoxylate von Fettsäuren, bevorzugt Polyethylenglykolester, Polypropylenglykolester, Polyethylenpolypropylenglykolester, Ethoxylate und/oder Propoxylate der Linolsäure, Linolensäure, Ölsäure, Arachidonsäure, besonders bevorzugt Ölsäureethoxylate. Alternativ können auch Polysiloxane verwendet werden. Salze von Fettsäuren mit Aminen, z.B. ölsaures Diethylamin, stearinsaures Diethanolamin, ricinolsaures Diethanolamin, Salze von Sulfonsäuren, z.B. Alkali- oder Ammoniumsalze von Dodecylbenzol- oder Dinaphthylmethandisulfonsäure sind ebenfalls bevorzugt.

Die sulfonierten Fettsäuren können bevorzugt als wässrige Lösungen, beispielsweise als 50%-ige Lösung eingesetzt werden. Typische bekannte Produkte sind Zusatzmittel SV und SM von Rheinchemie, sowie als nicht wässriger Emulgator Zusatzmittel WM von Rheinchemie.

Das Verfahren zur Herstellung der zelligen PUR-Gießelastomere wird in Gegenwart von Wasser durchgeführt. Das Wasser wirkt sowohl als Vernetzer unter Bildung von Harnstoffgruppen als auch aufgrund der Reaktion mit Isocyanatgruppen unter Bildung von Kohlendioxid als Treibmittel. Die Wassermengen, die zweckmäßigerweise verwendet werden können, betragen 0,01 bis 5 Gew.-%, vorzugsweise 0,3 bis 3,0 Gew.-%, bezogen auf das Gewicht der Polyolkomponente. Das Wasser kann vollständig oder teilweise in Form der wässrigen Lösungen der sulfonierten Fettsäuren eingesetzt werden.

Die Katalysatoren können einzeln wie auch in Abmischung miteinander zugegeben werden. Vorzugsweise sind dies metallorganische Verbindungen, wie Zinn-(II)-Salze von organischen Carbonsäuren, z. B. Zinn-(II)-dioctoat, Zinn-(II)-dilaurat, Dibutylzinndiacetat und Dibutylzinndilaurat und tertiäre Amine wie Tetramethyl-ethylendiamin, N-Methylmorpholin, Diethylbenzylamin, Triethylamin, Dimethylcyclohexylamin, Diazabicyclooctan, N,N'-Dimethylpiperazin, N-Methyl-N'-(4-N-Dimethylamino-)butylpiperazin, N,N,N',N" ,N" - Pentamethyldiethylentriamin oder ähnliche. Weiterhin kommen als Katalysatoren in Betracht: Amidine, wie z.B. 2,3-Dimethyl-3,4,5,6-tetrahydropyrimidin, Tris-(dialkyIamino-alkyl)-s-hexahydrotriazine, insbesondere Tris-(N,N-dimethylamino-propyl)-s-hexahydrotriazin, Tetraalkylammoniumhydroxide, wie z.B. Tetramethylammoniumhydroxid, Alkalihydroxide, wie z.B. Natriumhydroxid, und Alkalialkoholate, wie z.B. Natriummethylat und Kaliumisopropylat, sowie Alkalisalze von langkettigen Fettsäuren mit 10 bis 20 C-Atomen und gegebenenfalls seitenständigen OH Gruppen. Je nach einzustellender Reaktivität gelangen die Katalysatoren in Mengen von 0,001 bis 0,5 Gew.-%, bezogen auf die Isocyanatkomponente zur Anwendung.

Weitere Gegenstände der vorliegenden Erfindung sind ein Verfahren zur Herstellung eines Elastomers, bei dem mindestens eine erfindungsgemäße Zusammensetzung gegebenenfalls unter Erwärmen gehärtet wird sowie ein Elastomer, erhalten oder erhältlich durch, gegebenenfalls unter Erwärmen erfolgender Aushärtung der erfindungsgemäßen Zusammensetzung.

Die erfindungsgemäßen Polyurethane, Elastomere oder Formkörper unterscheiden sich von den im Stand der Technik bekannten auf monomeren bei Raumtemperatur festen Diisocyanaten, bevorzugt auf 1,5-Naphthalindiisocyanat basierenden Produkten dadurch, dass durch das erfindungsgemäße Verfahren auch die Kontamination des aus dem Rückstand zurückgewonnenen, monomeren bei Raumtemperatur festen Diisocyanats mit diesen Hilfsstoffen minimiert oder gar gänzlich vermieden werden kann.

Verwendung finden derartige zellige PUR-Gießelastomere, auch als Formkörper bezeichnet, als Dämpfungselemente im Fahrzeugbau, beispielsweise im Automobilbau, z. B. als Zusatzfedern, Anschlagpuffer, Querlenkerlager, Hinterachsenfahrschemellager, Stabilisator-Lager, Längsstreben-Lager, Federbein-Stützlager, Stoßdämpferlager, Lager für Dreieckslenker und als auf der Felge befindliches Notrad, das beispielsweise bei einem Reifenschaden bewirkt, dass das Fahrzeug auf dem zelligen Elastomer fährt und steuerbar bleibt. Die massiven Gießelastomere können für Rollen, Räder und Walzen, Rakel, Siebe oder Hydrozyklone verwendet werden.

### Beispiel 1

Ein Rückstand aus einer Destillation von 1,5-Naphthalindiisocyanat welches durch Phosgenierung von 1,5-Naphthalindiamin hergestellt wurde, wurde auf eine Kofler-Heizbank aufgetragen. Der Rückstand enthielt noch ca. 75 Gew.-% monomeres 1,5-Naphthalindiisocyanat (Bestimmt als Flächenprozent per GPC nach der DIN 55672-1:2007-08). Die Kofler-Heizbank wurde bei 100 bis 250 °C betrieben. Bei 250 °C erfolgte eine rasche Versprödung innerhalb von 2 Minuten und der Rückstand konnte als feines Pulver abgekratzt werden. Die Versprödung setzte sich im Folgenden auch in Bereichen tieferer Temperaturen fort und nach ca. 3 Stunden waren auch Bereiche bei 160 °C versprödet und ließen sich in Form von Flakes abkratzen. Unterhalb von 130 °C erfolgte keine Versprödung mehr.

### Beispiel 2

Ein Rückstand aus einer Destillation von 1,5-Naphthalindiisocyanat welches durch Phosgenierung von 1,5-Naphthalindiamin hergestellt wurde, wurde in einer Labor-Destillationsapparatur unter Vakuumbedingungen destilliert. Die Destillationsapparatur war mit einem Drehmomentmessrührer ausgestattet und das Drehmoment wurde im Laufe des Versuchs beobachtet. Der Druck im Inneren der Destillationsapparatur betrug 2 mbar und die Temperatur im Sumpf betrug zum Ende der Destillation ca. 260 °C. Nach ca. 7 Minuten Destillationsdauer kam es zu einem kurzen leichten Anstieg des Drehmoments bis auf 10 Ncm. Im folgenden Verlauf fiel das gemessene Drehmoment unter Ausbildung eines spröden Feststoffs wieder auf Werte <1 Ncm ab. Nach 60 Minuten wurde der Versuch beendet.

### Beispiel 3

Ein Rückstand aus einer Destillation von Toluylendiisocyanat welches durch Phosgenierung Toluylendiamin in der Gasphase hergestellt wurde, wurde in einer Labor-Destillationsapparatur unter Vakuumbedingungen destilliert. Die Destillationsapparatur war mit einem Drehmomentmessrührer ausgestattet und das Drehmoment wurde im Laufe des Versuchs beobachtet. Der Druck im Inneren der Destillationsapparatur betrug 100 mbar und die Temperatur im Sumpf betrug zum Ende der Destillation ca. 260 °C. Nach ca. 15 Minuten Destillationsdauer kam es zu einem stetigen Anstieg des Drehmoments. Nach 90 Minuten wurde der Versuch beendet. Das Drehmoment war auf 55 Ncm angestiegen und im Sumpf verblieb eine hochviskose, zähe Masse. Eine Versprödung wurde nicht beobachtet.

## Patentansprüche

1. Verfahren zur Rückgewinnung von monomeren bei Raumtemperatur festen Diisocyanaten aus einem Destillationsrückstand, umfassend die folgenden Schritte:
(i) Bereitstellen mindestens eines bei Raumtemperatur feste Diisocyanate enthaltenden Rückstands und
(ii) Trennung des Rückstands in mindestens einem Knetertrockner, Schaufeltrockner und/oder Walzentrockner in Gegenwart von weniger als 2 Gew.-% Bitumen, bezogen auf die Masse des in Schritt (i) bereitgestellten Rückstands, in einen gasförmigen Teil, enthaltend monomeres bei Raumtemperatur festes Diisocyanat, und einen spröden, an bei Raumtemperatur festem Diisocyanat abgereicherten Rückstand.

2. Verfahren gemäß Anspruch 1, **dadurch gekennzeichnet, dass** der Knetertrockner, Schaufeltrockner und/oder Walzentrockner ohne Kühlzone ausgeführt ist.

3. Verfahren Anspruche 1 oder 2, **dadurch gekennzeichnet, dass** die Trennung des Rückstandes in Schritt (ii) in mindestens einem Knetertrockner und/oder mindestens einem Walzentrockner und besonders bevorzugt in einem Walzentrockner erfolgt.

4. Verfahren gemäß einem der Ansprüche 1 bis 3, **dadurch gekennzeichnet, dass** die Trennung in Schritt (ii) in Gegenwart von ≤ 1 Gew.-% Bitumen, bevorzugt in Abwesenheit von Bitumen erfolgt.

5. Verfahren gemäß einem der Ansprüche 1 bis 4, **dadurch gekennzeichnet, dass** die Temperatur in Schritt (ii) bei ≥ 130 °C bis ≤ 270 °C, bevorzugt bei ≥ 140°C bis ≤ 200 °C und besonders bevorzugt bei ≥ 150 °C bis ≤ 190 °C liegt.

6. Verfahren gemäß einem der Ansprüche 1 bis 5, **dadurch gekennzeichnet, dass** der Druck in Schritt (ii) bei ≥ 1 mbar bis ≤ 1020 mbar, bevorzugt bei ≥ 1 mbar bis ≤ 25 mbar und besonders bevorzugt bei ≥ 1 mbar bis ≤ 10 mbar liegt.

7. Verfahren gemäß einem der Ansprüche 1 bis 6, **dadurch gekennzeichnet, dass** das bei Raumtemperatur feste Diisocyanat 1,5-Naphthalindiisocyanat, 1,8-Naphthalindiisocyanat oder 1,4-Phenylendiisocyanat, bevorzugt 1,5-Naphthalindiisocyanat oder 1,4-Phenylendiisocyanat und besonders bevorzugt 1,5-Naphthalindiisocyanat ist.

8. Verfahren gemäß einem der Ansprüche 1 bis 7, **dadurch gekennzeichnet, dass** der Rückstand bei der Trennung in Schritt (ii) eine mittlere Verweilzeit von ≥ 0,5 Minuten bis ≤ 4 Stunden Minuten, bevorzugt von ≥ 0,5 Minuten bis ≤ 60 Minuten, besonders bevorzugt von ≥ 0,5 bis ≤ 15 Minuten und ganz besonders bevorzugt von ≥ 1 bis ≤ 10 Minuten in dem mindestens einen Knetertrockner, Schaufeltrockner und/oder Walzentrockner aufweist.

9. Verfahren gemäß einem der Ansprüche 1 bis 8, **dadurch gekennzeichnet, dass** der in Schritt (ii) erhaltene, spröde Rückstand halbkontinuierlich ausgetragen wird.

10. Verfahren gemäß einem der Ansprüche 1 bis 9, **dadurch gekennzeichnet, dass** der in Schritt (ii) erhaltene, spröde Rückstand ≤ 5 Gew.-%, bevorzugt ≤ 2 Gew.-%, besonders bevorzugt ≤ 0,2 Gew.-% und ganz besonders bevorzugt < 0,1 Gew.-% an monomerem, bei Raumtemperatur festem Diisocyanat bezogen auf die Gesamtmasse des Rückstands enthält.

11. Verfahren gemäß einem der Ansprüche 1 bis 10, **dadurch gekennzeichnet, dass** der in Schritt (i) bereitgestellte bei Raumtemperatur feste Diisocyanate enthaltende Rückstand aus der Destillation eines durch Phosgenierung der entsprechenden Diamine hergestellten Diisocyanats stammt.

12. Monomeres bei Raumtemperatur festes Diisocyanat, erhalten oder erhältlich nach einem Verfahren gemäß einem der Ansprüche 1 bis 11.

13. Verwendung eines Knetertrockners, Schaufeltrockners und/oder Walzentrockners zur Minimierung des Hilfsstoffeinsatzes bei der Versprödung eines bei Raumtemperatur feste Diisocyanate enthaltenden Rückstands.

14. Zusammensetzung enthaltend mindestens ein monomeres bei Raumtemperatur festes Diisocyanat gemäß Anspruch 12 und mindestens eine NCO-reaktive Verbindung, bevorzugt mindestens ein Polyesterpolyol.

15. Elastomer, erhalten oder erhältlich durch, gegebenenfalls unter Erwärmen erfolgender Aushärtung einer Zusammensetzung gemäß Anspruch 14.
